# DEMANDE DE BREVET EUROPEEN

(11) **EP 3 453 383 A1**
(43) Date de publication de la demande: **13.03.2019**
(21) Numéro de dépôt: 18193257.5
(22) Date de dépôt: 07.09.2018
(51) Int. Cl.: A61K 9/00, A61K 9/20, A61K 31/202, A23L 33/115

(54) **PROCÉDÉ DE PRÉPARATION D'UN COMPRIMÉ POUR ADMINISTRATION PERLINGUALE ET/OU SUBLINGUALE COMPRENANT UNE OU PLUSIEURS HUILES ESSENTIELLES**

(30) Priorité: 08.09.2017 FR 1758300
(71) Demandeur: Mercure Pharma Consulting, 74940 Annecy le Vieux (FR)
(72) Inventeur: ILIOZER, Vreje, 07130 SAINT-PERAY (FR); CHIANEA, Stephan, 01700 BEYNOST (FR)
(74) Mandataire: Casalonga

(57) **Abrégé**

La présente invention concerne un procédé de préparation d'un comprimé pour administration perlinguale et/ou sublinguale comprenant une ou plusieurs huiles essentielles.

La présente invention porte en outre sur un comprimé obtenu à partir dudit procédé, et de son utilisation comme complément alimentaire.

La présente invention concerne en outre un composé destiné à être utilisé comme médicament.

## Description

La présente invention concerne un procédé de préparation d'un comprimé pour administration perlinguale et/ou sublinguale comprenant une ou plusieurs huiles essentielles.

La présente invention porte en outre sur un comprimé obtenu à partir dudit procédé, et de son utilisation comme complément alimentaire.

La présente invention concerne également un comprimé destiné à être utilisé comme médicament.

Les huiles essentielles sont utilisées en médecine depuis des siècles et sont traditionnellement administrées par voie sublinguale et/ou perlinguale lorsqu'une action systémique sur l'organisme est souhaitée. En effet, ces voies d'administrations sont particulièrement intéressantes, puisqu'elles évitent le premier passage hépatique et garantissent donc une biodisponibilité optimale des actifs présents dans les huiles essentielles.

Cependant, compte tenu de leurs très fortes concentrations et de leur potentielle causticité, une utilisation pure de ces huiles essentielles n'est pas envisageable. Elles sont donc traditionnellement diluées de façon extemporanée sur un support, tel qu'un comprimé neutre (le plus souvent à base de lactose), une cuillère d'huile végétale, une cuillère de miel, voire un morceau de pain.

Ces méthodes d'utilisation présentent cependant plusieurs inconvénients. Elles sont premièrement très peu pratiques à utiliser, ce qui entraîne le plus souvent une mauvaise observance par les patients, notamment lorsque ces derniers sont amenés à se déplacer. Ces méthodes requièrent entre outre une bonne rigueur de la part du patient au moment du dosage de l'huile essentielle. En effet, le mésusage, et en particulier le surdosage de l'huile essentielle peut s'avérer catastrophique au vu de la puissance des actifs naturels contenus dans ces huiles.

Pour pallier à ces inconvénients, il existe, sur le marché, des préparations commerciales prêtes à l'emploi contenant une quantité précise et diluée d'une ou plusieurs huiles essentielles. Il s'agit de gélules ou de capsules molles contenant une ou plusieurs huiles essentielles diluées dans une solution lipidique.

Contrairement à l'utilisation traditionnelle, ces préparations sont administrées par voie orale entraînant une dégradation plus importante des actifs contenus dans les huiles essentielles lors du passage de la barrière digestive et du premier passage hépatique. La biodisponibilité est alors plus faible, ce qui se traduit, in fine, par une activité systémique amoindrie par rapport à l'utilisation traditionnelle.

Les huiles essentielles sont des composés liquides et ne peuvent être intégrées dans des comprimés solides que par granulation humide. Or, les actifs de ces huiles sont fragiles, et notamment volatiles. Ils ont tendance à s'altérer, voire s'évaporer, durant les phases de séchage indissociables des procédés de granulation.

Ainsi, il existe un réel besoin de développer un procédé de préparation de comprimés, et notamment de comprimés à sucer, agréables en bouche et permettant l'administration sécurisée sublinguale et/ou perlinguale des huiles essentielles.

La répartition de la teneur en huiles essentielles dans les comprimés doit en outre être homogène et les principes actifs contenus dans ces huiles ne doivent pas s'altérer durant tout le procédé de fabrication.

Les comprimés ainsi obtenus doivent permettre aux patients de mieux suivre leurs traitements et d'éviter tout risque de mésusage, tout en conservant l'efficacité optimale des huiles essentielles.

La demanderesse a découvert qu'un procédé de préparation d'un comprimé, et notamment d'un comprimé à sucer, pour administration perlinguale et/ou sublinguale comprenant une étape d'imprégnation d'une ou plusieurs huiles essentielles sur du phosphate de calcium anhydre permettait d'atteindre les objectifs cités ci-avant.

La présente invention a notamment pour objet un procédé de préparation d'un comprimé pour administration perlinguale et/ou sublinguale comprenant les étapes successives suivantes :
(a) une étape d'imprégnation d'une ou plusieurs huiles essentielles sur du phosphate de calcium anhydre,
(b) une étape de préparation d'une composition comprenant le phosphate de calcium imprégné obtenu à l'étape (a), du stéarate de magnésium, de la silice, du sorbitol, un mélange d'édulcorants contenant du glycoside de stéviol, du sucralose et du saccharinate de sodium, et une cellulose microcristalline, et
(c) une étape de compression directe de la composition obtenue à l'étape (b) ;
le mélange d'édulcorants étant présent en une quantité totale allant de 0,05 à 1% en poids, par rapport au poids total de la composition finale.

Par « composition finale », on entend, au sens de la présente invention, le comprimé obtenu à l'issue du procédé de l'invention.

Le procédé selon la présente invention permet d'obtenir des comprimés présentant une dureté suffisante et une répartition homogène de l'huile essentielle au sein du comprimé.

En outre, contrairement aux procédés de granulation classique, le procédé de l'invention ne nécessite pas d'étape de séchage. L'étape d'imprégnation permet ainsi d'éviter l'altération des principes actifs contenus dans les huiles essentielles. Les comprimés ainsi obtenus permettent l'administration d'une dose contrôlée d'actifs sans risque de mésusage, ni de surdosage. Par ailleurs, l'administration sublinguale et/ou perlinguale évite les sucs gastriques et le passage hépatique, permettant ainsi aux actifs d'atteindre directement leurs cibles.

Par ailleurs, le mélange d'édulcorants particuliers mis en oeuvre dans le procédé de l'invention permet d'adoucir et/ou de masquer le goût des autres ingrédients, et notamment celui des huiles essentielles. Le comprimé obtenu est ainsi plus agréable en bouche, et peut être consommé par une personne diabétique.

Ce mélange d'édulcorants et sa teneur respectent en outre la réglementation européenne en matière de compléments alimentaires.

La présente invention a donc également pour objet un comprimé obtenu à partir du procédé de préparation tel que mentionné précédemment.

La présente invention concerne également l'utilisation dudit comprimé comme complément alimentaire.

La présente invention porte en outre sur un comprimé, tel que défini précédemment, destiné à être utilisé comme médicament.

Selon la ou les huiles essentielles utilisées, le comprimé selon l'invention peut avantageusement apaiser les voies respiratoires, conserver un bon maintien des systèmes immunitaire et respiratoire, soulager les douleurs, contribuer à la relaxation, aider à maintenir un sommeil sain, aider à réduire la fatigue, favoriser le confort digestif, exercer une action favorable sur la digestion ou réduire la sensation d'inconfort lié au transport.

D'autres objets, caractéristiques, aspects et avantages de l'invention apparaîtront encore plus clairement à la lecture de la description et des exemples qui suivent.

Dans ce qui va suivre, et à moins d'une autre indication, les bornes d'un domaine de valeurs sont comprises dans ce domaine, notamment dans les expressions « compris entre » et « allant de ... à ... ».

Par ailleurs, les expressions « au moins un », « au moins deux », et « au moins » utilisées dans la présente description sont respectivement équivalentes aux expressions « un ou plusieurs », « deux ou plusieurs » et « supérieur ou égal ».

### Imprégnation d'une ou plusieurs huiles essentielles sur du phosphate de calcium anhydre

Le procédé selon la présente invention comprend une étape d'imprégnation d'une ou plusieurs huiles essentielles sur du phosphate de calcium anhydre.

Par « phosphate de calcium imprégné », on entend, au sens de la présente invention, du phosphate de calcium anhydre imprégné par une ou plusieurs huiles essentielles. En d'autres termes, le phosphate de calcium imprégné est obtenu à l'issu de l'étape (a).

La quantité totale de phosphate de calcium anhydre, selon la présente invention, va de préférence de 1 à 15% en poids, et plus préférentiellement de 5 à 10% en poids, par rapport au poids total de la composition finale.

Les huiles essentielles sont de préférence des produits naturels extraits de plantes aromatiques.

Les huiles essentielles utilisables selon la présente invention sont de préférence extraites, par entraînement à la vapeur, à partir des fleurs, des feuilles, des racines, de l'écorce, des sommités fleuries, des aiguilles et parties aériennes, des fruits ou encore des graines.

A titre d'huiles essentielles, on peut également utiliser les essences aromatiques issues de la pression à froid de plantes aromatiques, telles que les essences issues des écorces d'agrumes comme l'essence de citron ou l'essence d'orange.

De préférence, la ou les huiles essentielles, utilisées dans la présente invention, sont choisies parmi les huiles essentielles de ravintsara, de thym, de pin sylvestre, de romarin, de menthe, de lavande, de petit grain de bigarade, d'eucalyptus, d'origan, de marjolaine, de laurier, d'arbre à thé, d'hélichryse, de cannelle, de poivre, de gingembre, de coriandre, de fenouil, de cyprès, de camomille, d'estragon et d'origan, les essences de citron, d'orange et de mandarine, et leurs mélanges.

La quantité totale de la ou des huiles essentielles, selon la présente invention, va de préférence de 0,1 à 10% en poids, et plus préférentiellement de 0,5 à 5% en poids, par rapport au poids total de la composition finale.

Le rapport pondéral entre la quantité totale de la ou des huiles essentielles et la quantité totale de phosphate de calcium anhydre, mis en oeuvre dans le procédé selon la présente invention, est de préférence inférieur ou égal à 1. Plus préférentiellement, ce rapport pondéral va de 0,05 à 1, et mieux encore de 0,1 à 0,8.

L'étape d'imprégnation selon le procédé de l'invention comprend de préférence une étape de mouillage du phosphate de calcium anhydre avec la ou les huiles essentielles, suivie d'une étape de mélange.

De préférence, la durée de l'étape de mouillage va de 1 à 3 minutes, et plus préférentiellement de 1 minute 30 secondes à 2 minutes 30 secondes. La durée particulière de l'étape de mouillage permet d'obtenir un comprimé de meilleure qualité, plus homogène et moins collant.

De préférence, la durée de l'étape de mélange va de 20 secondes à 5 minutes, et plus préférentiellement de 30 secondes à 2 minutes. La durée particulière de cette étape de mélange permet d'obtenir un comprimé plus homogène et évite l'évaporation des huiles essentielles.

### Préparation de la composition

Le procédé selon la présente invention comprend en outre une étape de préparation d'une composition comprenant le phosphate de calcium imprégné obtenu à l'étape (a), du stéarate de magnésium, de la silice, du sorbitol, un mélange d'édulcorants contenant du glycoside de stéviol, du sucralose et du saccharinate de sodium, et une cellulose microcristalline.

De préférence le stéarate de magnésium et la silice sont préalablement tamisés avant d'être mélangés au phosphate de calcium imprégné. En d'autres termes, le procédé selon la présente invention, peut éventuellement comprendre en outre une étape de tamisage du stéarate de magnésium et de la silice, préalable à l'étape de préparation de la composition. Lorsqu'elle est présente, cette étape de tamisage est effectuée avant l'étape de préparation de la composition.

Le tamis utilisé lors de cette étape de tamisage comprend de préférence des mailles allant de 0,5 à 5mm.

La quantité totale du stéarate de magnésium et de la silice, selon la présente invention, va de préférence de 0,1 à 5% en poids, et plus préférentiellement de 0,5 à 3% en poids, par rapport au poids total de la composition finale.

La composition, préparée lors de l'étape (b) du procédé selon l'invention, comprend en outre du sorbitol.

La quantité totale de sorbitol, selon la présente invention, va de préférence de 60 à 80% en poids, et plus préférentiellement de 65 à 75% en poids, par rapport au poids total de la composition finale.

La composition, préparée lors de l'étape (b) du procédé selon l'invention, comprend en outre une cellulose microcristalline.

La quantité totale de la cellulose microcristalline, selon la présente invention, va de préférence de 12 à 27% en poids, et plus préférentiellement de 15 à 25% en poids, par rapport au poids total de la composition finale.

La composition, préparée lors de l'étape (b) du procédé selon la présente invention, comprend en outre un mélange d'édulcorants contenant du glycoside de stéviol, du sucralose et du saccharinate de sodium.

La quantité totale du mélange d'édulcorants va de préférence de 0,1 à 0,5% en poids, par rapport au poids total de la composition finale.

La composition, préparée selon le procédé de la présente invention, peut éventuellement comprendre en outre un ou plusieurs composés additionnels.

A titre de composés additionnels utilisables conformément à l'invention, on peut citer les colorants, les arômes, les agents désintégrants et les agents conservateurs.

Bien entendu, l'homme de l'art veillera à choisir ce ou ces éventuels composés additionnels de manière telle que les propriétés avantageuses attachées intrinsèquement au comprimé final obtenu à l'issue du procédé de préparation selon l'invention ne soient pas, ou substantiellement pas, altérées par la ou les adjonctions envisagées.

Selon un mode de réalisation préféré de l'invention, la composition préparée à l'étape (b) peut être obtenue à partir du mélange de deux compositions. Plus particulièrement, l'étape (b), du procédé selon l'invention, peut éventuellement comprendre une étape de préparation d'une composition (A) comprenant le phosphate de calcium imprégné obtenu à l'étape (a), du stéarate de magnésium et de la silice, et une étape de préparation d'une composition (B) comprenant du sorbitol, un mélange d'édulcorants contenant du glycoside de stéviol, du sucralose et du saccharinate de sodium, et une cellulose microcristalline, suivie d'une étape de mélange des compositions (A) et (B).

En d'autres termes, selon ce mode de réalisation, le procédé comprend les étapes successives suivantes :
(a) une étape d'imprégnation d'une ou plusieurs huiles essentielles sur du phosphate de calcium anhydre,
(b) une étape de préparation d'une composition (A) comprenant le phosphate de calcium imprégné obtenu à l'étape (a), du stéarate de magnésium et de la silice,
(c) une étape de préparation d'une composition (B) comprenant du sorbitol, un mélange d'édulcorants contenant du glycoside de stéviol, du sucralose et du saccharinate de sodium, et une cellulose micro cristalline,
(d) une étape de mélange des compositions (A) et (B) obtenues aux étapes précédentes (b) et (c), et
(e) une étape de compression du mélange obtenu à l'étape (d) ;
le mélange d'édulcorants étant présent en une quantité totale allant de 0,05 à 1% en poids, par rapport au poids total de la composition finale.

Lorsqu'elle est présente, la quantité totale de composition (A) va de préférence de 5 à 20% en poids, et plus préférentiellement de 8 à 15% en poids, par rapport au poids total de la composition finale.

Lorsqu'elle est présente la quantité totale de composition (B) va de préférence de 80 à 95% en poids, et plus préférentiellement de 85 à 92% en poids, par rapport au poids total de la composition finale.

Selon un autre mode de réalisation de l'invention, l'étape (b) du procédé selon l'invention peut comprendre une étape de préparation d'une composition (C) comprenant de la silice, un mélange d'édulcorants contenant du glycoside de stéviol, du sucralose et du saccharinate de sodium, et une cellulose microcristalline, et une étape de mélange du phosphate de calcium imprégné obtenu à l'étape (a) avec la composition (C), du sorbitol, et du stéarate de magnésium.

En d'autres termes, selon ce mode de réalisation, le procédé peut comprendre les étapes suivantes :
(a) une étape d'imprégnation d'une ou plusieurs huiles essentielles sur du phosphate de calcium anhydre,
(b) une étape de préparation d'une composition (C) comprenant de la silice, un mélange d'édulcorants contenant du glycoside de stéviol, du sucralose et du saccharinate de sodium, et une cellulose micro cristalline,
(c) une étape de mélange du phosphate de calcium imprégné obtenu à l'étape (a) avec la composition (C) obtenue à l'étape (b), du sorbitol, et du stéarate de magnésium, et
(d) une étape de compression du mélange obtenu à l'étape (c) ;
le mélange d'édulcorants étant présent en une quantité totale allant de 0,05 à 1% en poids, par rapport au poids total de la composition finale.

La présente invention concerne également un comprimé obtenu à partir du procédé de préparation tel que défini précédemment.

De préférence, le comprimé de l'invention est un comprimé à sucer.

Un autre objet de la présente invention concerne l'utilisation de ce composé comme complément alimentaire.

La présente invention a en outre pour objet un comprimé, tel que défini précédemment, destiné à être utilisé comme médicament.

Les exemples suivants servent à illustrer l'invention sans toutefois présenter un caractère limitatif.

### EXEMPLES

### Comprimés selon l'invention

Les comprimés 1 à 6 ont été obtenus à partir du procédé selon la présente invention. Ils ont été préparés à partir des ingrédients indiqués dans les tableaux ci-dessous et dont les quantités sont exprimées en pourcentage de matière active, par rapport au poids total de la composition.

| | Comprimé 1 |
|---|---|
| Sorbitol | 69,23 |
| Cellulose microcristalline | 18,27 |
| Phosphate de calcium anhydre (Fujicalin) | 7,28 |
| Huile essentielle de pin sylvestre (*Pinus sylvestris L.,* aiguille) | 1,28 |
| Huile essentielle de thym à linalol (*Thimus vulgaris L.,* partie aérienne fleurie) | 1,28 |
| Stéarate de magnésium | 1,00 |
| Huile essentielle de ravintsara (*Cinnamomum camphora L.,* feuille) | 0,77 |
| Silice colloidale | 0,50 |
| Sulfate de zinc monohydraté | 0,05 |
| Glycoside de stéviol | 0,20 |
| Sucralose | 0,08 |
| Saccharinate de sodium | 0,05 |

L'utilisation du comprimé 1 permet de maintenir le bon fonctionnement des systèmes respiratoire et immunitaire.

| | Comprimé 2 |
|---|---|
| Sorbitol | 69,23 |
| Cellulose microcristalline | 17,81 |
| Phosphate de calcium anhydre (Fujicalin) | 7,28 |
| Huile essentielle d'eucalyptus globuleux (*Eucalyptus globulus Labill.,* feuilles et tiges) | 1,54 |
| Huile essentielle de menthe citronnée (*Mentha citrata,* partie aérienne fleurie) | 1,54 |
| Stéarate de magnésium | 1,00 |
| Huile essentielle de thym à linalol (*Thimus vulgaris L.,* partie aérienne fleurie) | 0,77 |
| Silice colloidale | 0,50 |
| Glycoside de stéviol | 0,20 |
| Sucralose | 0,08 |
| Saccharinate de sodium | 0,05 |

L'utilisation du comprimé 2 permet de maintenir le bon fonctionnement du système respiratoire. Il permet en outre d'apaiser la gorge, le pharynx et les cordes vocales.

| | Comprimé 3 |
|---|---|
| Sorbitol | 69,23 |
| Cellulose microcristalline | 17,81 |
| Phosphate de calcium anhydre (Fujicalin) | 7,28 |
| Huile essentielle d'eucalyptus globuleux (*Eucalyptus globulus Labill.,* feuilles et tiges) | 1,54 |
| Huile essentielle de romarin à camphre (*Rosmarinus officinalis L.,* rameaux et sommités fleuries) | 1,54 |
| Stéarate de magnésium | 1,00 |
| Huile essentielle de ravintsara (*Cinnamomum camphora L.,* feuille) | 0,77 |
| Silice colloidale | 0,50 |
| Glycoside de stéviol | 0,20 |
| Sucralose | 0,08 |
| Saccharinate de sodium | 0,05 |

L'utilisation du comprimé 3 apaise les voies respiratoires.

| | Comprimé 4 |
|---|---|
| Sorbitol | 69,23 |
| Cellulose microcristalline | 18,58 |
| Phosphate de calcium anhydre (Fujicalin) | 7,28 |
| Huile essentielle de fenouil (*Foeniculum vulgare,* semence) | 1,54 |
| Stéarate de magnésium | 1,00 |
| Huile essentielle de cyprès (*Cupressus sempervirens L.,* rameau) | 0,77 |
| Huile essentielle de petit grain de bigarade (*Citrus aurantium* (*var amara L.),* péricarpe) | 0,77 |
| Silice colloidale | 0,50 |
| Glycoside de stéviol | 0,20 |
| Sucralose | 0,08 |
| Saccharinate de sodium | 0,05 |

L'utilisation du comprimé 4 apaise les voies respiratoires supérieures.

| | Comprimé 5 |
|---|---|
| Sorbitol | 69,23 |
| Cellulose microcristalline | 19,30 |
| Phosphate de calcium anhydre (Fujicalin) | 7,28 |
| Stéarate de magnésium | 1,00 |
| Huile essentielle de menthe poivrée (*Mentha piperita L.,* sommité fleurie) | 0,77 |
| Huile essentielle de camomille matriciare (*Matricaria chamomilla L.,* sommité fleurie) | 0,77 |
| Huile essentielle d'estragon *(Artemisia dracunculus L,* partie aérienne) | 0,77 |
| Sulfate de zinc monohydraté | 0,05 |
| Silice colloidale | 0,50 |
| Glycoside de stéviol | 0,20 |
| Sucralose | 0,08 |
| Saccharinate de sodium | 0,05 |

| | Comprimé 6 |
|---|---|
| Sorbitol | 69,23 |
| Cellulose microcristalline | 20,58 |
| Phosphate de calcium anhydre (Fujicalin) | 7,28 |
| Stéarate de magnésium | 1,00 |
| Huile essentielle de thym à linalol (*Thimus vulgaris L.,* partie aérienne fleurie) | 0,64 |
| Sulfate de zinc monohydraté (36% en poids) | 0,05 |
| Silice colloidale | 0,50 |
| Glycoside de stéviol | 0,20 |
| Huile essentielle d'origan (*Origanum vulgare L.,* plante) | 0,19 |
| Huile essentielle de cannelle (Cinnamomum zeylanicum Blume, écorce) | 0,19 |

| | |
|---|---|
| Sucralose | 0,08 |
| Saccharinate de sodium | 0,05 |

L'utilisation des comprimés 5 et 6 permet de maintenir un bon fonctionnement du système immunitaire.

## Revendications

1. Procédé de préparation d'un comprimé pour administration perlinguale et/ou sublinguale comprenant les étapes successives suivantes :
(a) une étape d'imprégnation d'une ou plusieurs huiles essentielles sur du phosphate de calcium anhydre,
(b) une étape de préparation d'une composition comprenant le phosphate de calcium imprégné obtenu à l'étape (a), du stéarate de magnésium, de la silice, du sorbitol, un mélange d'édulcorants contenant du glycoside de stéviol, du sucralose et du saccharinate de sodium, et une cellulose microcristalline, et
(c) une étape de compression directe de la composition obtenue à l'étape (b) ;
le mélange d'édulcorants étant présent en une quantité totale allant de 0,05 à 1% en poids, par rapport au poids total de la composition finale.

2. Procédé selon la revendication 1, **caractérisé en ce que** la quantité totale de phosphate de calcium anhydre va de 1 à 15% en poids, et de préférence de 5 à 10% en poids, par rapport au poids total de la composition finale.

3. Procédé selon l'une quelconque des revendications précédentes, **caractérisé en ce que** la ou les huiles essentielles sont choisies parmi les huiles essentielles de ravintsara, de thym, de pin sylvestre, de romarin, de menthe, de lavande, de petit grain de bigarade, d'eucalyptus, d'origan, de marjolaine, de laurier, d'arbre à thé, d'hélichryse, de cannelle, de poivre, de gingembre, de coriandre, de fenouil, de cyprès, de camomille, d'estragon et d'origan, les essences de citron, d'orange et de mandarine, et leurs mélanges.

4. Procédé selon l'une quelconque des revendications précédentes, **caractérisé en ce que** la quantité totale de la ou des huiles essentielles va de 0,1 à 10% en poids, et de préférence de 0,5 à 5% en poids, par rapport au poids total de la composition finale.

5. Procédé selon l'une quelconque des revendications précédentes, **caractérisé en ce que** le rapport pondéral entre la quantité totale de la ou des huiles essentielles et la quantité totale de phosphate de calcium anhydre est inférieur ou égal à 1, va de préférence de 0,05 à 1, et plus préférentiellement de 0,1 à 0,8.

6. Procédé selon l'une quelconque des revendications précédentes, **caractérisé en ce que** l'étape (a) d'imprégnation comprend une étape de mouillage du phosphate de calcium anhydre avec la ou les huiles essentielles, suivie d'une étape de mélange.

7. Procédé selon la revendication précédente, **caractérisé en ce que** la durée de l'étape de mouillage va de 1 à 3 minutes, et de préférence de 1 minute 30 secondes à 2 minutes 30 secondes.

8. Procédé selon l'une quelconque des revendications 6 ou 7, **caractérisé en ce que** la durée de l'étape de mélange va de 20 secondes à 5 minutes, et de préférence de 30 secondes à 2 minutes.

9. Procédé selon l'une quelconque des revendications précédentes, **caractérisé en ce que** la quantité totale du mélange d'édulcorants va de 0,1 à 0,5% en poids, par rapport au poids total de la composition finale.

10. Procédé selon l'une quelconque des revendications précédentes, **caractérisé en ce que** l'étape (b) comprend une étape de préparation d'une composition (A) comprenant le phosphate de calcium imprégné obtenu à l'étape (a), du stéarate de magnésium et de la silice, et une étape de préparation d'une composition (B) comprenant du sorbitol, un mélange d'édulcorants contenant du glycoside de stéviol, du sucralose et du saccharinate de sodium, et une cellulose microcristalline, suivie d'une étape de mélange des compositions (A) et (B).

11. Procédé selon l'une quelconque des revendications 1 à 9, **caractérisé en ce que** l'étape (b) comprend une étape de préparation d'une composition (C) comprenant de la silice, un mélange d'édulcorants contenant du glycoside de stéviol, du sucralose et du saccharinate de sodium, et une cellulose microcristalline, et une étape de mélange du phosphate de calcium imprégné obtenu à l'étape (a) avec la composition (C), du sorbitol, et du stéarate de magnésium.

12. Comprimé obtenu à partir d'un procédé de préparation selon l'une quelconque des revendications précédentes.

13. Utilisation d'un comprimé selon la revendication 12 comme complément alimentaire.

14. Comprimé selon la revendication 12 destiné à être utilisé comme médicament.
